(19) Europäisches Patentamt
European Patent Office
Office européen des brevets

(11) EP 0 764 155 B1

(12) **FASCICULE DE BREVET EUROPEEN**

(45) Date de publication et mention de la délivrance du brevet:
**26.08.1998 Bulletin 1998/35**

(21) Numéro de dépôt: **95922570.7**

(22) Date de dépôt: **07.06.1995**

(51) Int Cl.[6]: **C07D 305/14**, A61K 31/335

(86) Numéro de dépôt international:
**PCT/FR95/00736**

(87) Numéro de publication internationale:
**WO 95/33737 (14.12.1995 Gazette 1995/53)**

(54) **TAXOIDES, LEUR PREPARATION ET LES COMPOSITIONS PHARMACEUTIQUES QUI LES CONTIENNENT**

TAXOIDE, IHRE HERSTELLUNG UND DIESE ENTHALTENDE PHARMAZEUTISCHE ZUSAMMENSETZUNGEN

TAXOIDS, PREPARATION AND PHARMACEUTICAL COMPOSITIONS CONTAINING THEM

(84) Etats contractants désignés:
**AT BE CH DE DK ES FR GB GR IE IT LI LU NL PT SE**

(30) Priorité: **09.06.1994 FR 9407050**

(43) Date de publication de la demande:
**26.03.1997 Bulletin 1997/13**

(73) Titulaire: **RHONE-POULENC RORER S.A.**
**92160 Antony (FR)**

(72) Inventeurs:
- **BOUCHARD, Hervé**
  **F-94200 Ivry-sur-Seine (FR)**
- **BOURZAT, Jean-Dominique**
  **F-94300 Vincennes (FR)**
- **COMMERCON, Alain**
  **F-94400 Vitry-sur-Seine (FR)**

(74) Mandataire: **Le Pennec, Magali et al**
**RHONE-POULENC RORER SA,**
**Direction des Brevets,**
**20 Avenue Raymond Aron**
**92165 Antony Cédex (FR)**

(56) Documents cités:
**EP-A- 0 577 082**
**EP-A- 0 577 083**
**EP-A- 0 590 267**
**WO-A-93/06093**
**WO-A-94/13654**
**WO-A-95/33738**
**WO-A-95/33739**

- **CHEMICAL ABSTRACTS, vol. 119, no. 21, 22 Novembre 1993, Columbus, Ohio, US; abstract no. 226216n, & JOURNAL OF ORGANIC CHEMISTRY, vol.58, no.17, 1993, EASTON US pages 4520 - 4521 S. H. CHEN ET AL.**
- **CHEMICAL ABSTRACTS, vol. 120, no. 17, 25 Avril 1994, Columbus, Ohio, US; abstract no. 218230t, & TETRAHEDRON LETTERS, vol.35, no.1, 1994, OXFORD GB pages 41 - 44 S. H. CHEN ET AL.**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 764 155 B1

Printed by Jouve, 75001 PARIS (FR)

## Description

La présente invention concerne concerne de nouveaux taxoïdes de formule générale :

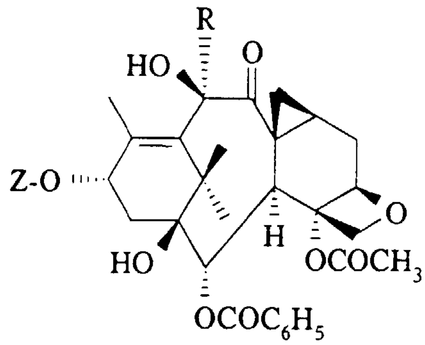

(I)

dans laquelle :

- R représente un radical alcoyle droit ou ramifié contenant 1 à 6 atomes de carbone, alcényle droit ou ramifié contenant 2 à 6 atomes de carbone, alcynyle droit ou ramifié contenant 2 à 6 atomes de carbone, cycloalcoyle contenant 3 à 6 atomes de carbone, cycloalcényle contenant 4 à 6 atomes de carbone, aryle, hétérocyclyle aromatique contenant 5 à 6 chaînons

Z représente un atome d'hydrogène ou un radical de formule générale :

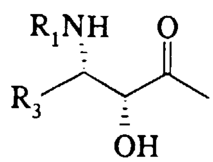

(II)

dans laquelle :

$R_1$ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical $R_2$-O-CO- dans lequel $R_2$ représente :

- un radical alcoyle contenant 1 à 8 àtomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,

$R_3$ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles.

De préférence les radicaux aryles pouvant être représentés par R et $R_3$ sont des radicaux phényles ou α- ou β-naphtyles éventuellement substitués par un ou plusieurs atomes ou radicaux choisis parmi les atomes d'halogène (fluor, chlore, brome, iode) et les radicaux alcoyles, alcényles, alcynyles, aryles, arylalcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, dialcoylcarbamoyle, cyano, nitro et trifluoro-méthyle, étant entendu que les radicaux alcoyles et les portions alcoyles des autres radicaux contiennent 1 à 4 atomes de carbone, que les radicaux alcényles et alcynyles contiennent 2 à 8 atomes de carbone et que les radicaux aryles sont des radicaux phényles ou α- ou β-naphtyles.

De préférence les radicaux hétérocycliques pouvant être représentés par R et $R_3$ sont des radicaux hétérocycliques aromatiques ayant 5 chaînons et contenant un ou plusieurs atomes, identiques ou différents, choisis parmi les atomes d'azote, d'oxygène ou de soufre, éventuellement substitués par un ou plusieurs substituants, identiques ou différents, choisis parmi les atomes d'halogène (fluor, chlore, brome, iode) et les radicaux alcoyles contenant 1 à 4 atomes de carbone, aryles contenant 6 à 10 atomes de carbone, alcoxy contenant 1 à 4 atomes de carbone, aryloxy contenant 6 à 10 atomes de carbone, amino, alcoylamino contenant 1 à 4 atomes de carbone, dialcoylamino dont chaque partie alcoyle contient 1 à 4 atomes de carbone, acylamino dont la partie acyle contient 1 à 4 atomes de carbone, alcoxycarbonylamino contenant 1 à 4 atomes de carbone, acyle contenant 1 à 4 atomes de carbone, arylcarbonyle dont la partie aryle contient 6 à 10 atomes de carbone, cyano, carboxy, carbamoyle, alcoylcarbamoyle dont la partie alcoyle contient 1 à 4 atomes de carbone, dialcoylcarbamoyle dont chaque partie alcoyle contient 1 à 4 atomes de carbone ou alcoxycarbonyle dont la partie alcoxy contient 1 à 4 atomes de carbone.

La demande de brevet EP 577083 décrit des taxoïdes porteurs en position 7,8 d'un cycle cyclopropyle et en position 10 d'un motif alkylcarbonyloxy, acyloxy, de l'hydrogène, d'un motif hydroxyle ou carbonyle alors que les dérivés de la présente invention sont porteurs en position 10 conjointement d'un groupe hydroxyle et d'un autre motif.

Plus particulièrement, la présente invention concerne les produits de formule générale (I) dans laquelle R représente un radical alcoyle contenant 1 à 4 atomes de carbone, Z représente un atome d'hydrogène ou un radical de formule générale (II) dans laquelle $R_1$ représente un radical benzoyle ou un radical $R_2$-O-CO- dans lequel $R_2$ représente un radical tert-butyle et $R_3$ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles (méthyle), alcoxy (méthoxy), dialcoylamino (diméthylamino), acylamino (acétylamino), alcoxycarbonylamino (tertbutoxycarbonylamino) ou trifluorométhyle ou un radical furyle-2 ou -3, thiényle-2 ou - 3 ou thiazolyle-2, -4 ou -5.

Plus particulièrement encore, la présente invention concerne les produits de formule générale (I) dans laquelle R représente un radical méthyle, Z représente un atome d'hydrogène ou un radical de formule générale (II) dans laquelle $R_1$ représente un radical benzoyle ou un radical $R_2$-O-CO- dans lequel $R_2$ représente un radical tert-butyle et $R_3$ représente un radical isobutyle, isobutényle, butényle, cyclohexyle, phényle, furyle-2, furyle-3, thiényle-2, thiényle-3, thiazolyle-2, thiazolyle-4 ou thiazolyle-5.

Les produits de formule générale (I) dans laquelle Z représente un radical de formule générale (II) présentent des propriétés antitumorales et antileucémiques remarquables.

Selon la présente invention, les produits de formule générale (I) dans laquelle R et Z sont définis comme précédemment peuvent être obtenus par action d'un dérivé organométallique de formule générale :

$$R\text{-}X \qquad \text{(III)}$$

dans laquelle R est défini commme précédemment et X représente un atome de métal tel qu'un atome de lithium ou un reste d'organo-magnésien (Mg-Y dans lequel Y représente un atome d'halogène) sur un produit de formule générale :

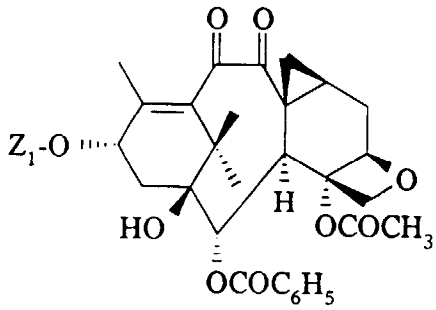

(IV)

dans laquelle $Z_1$ représente un atome d'hydrogène ou un radical de formule générale :

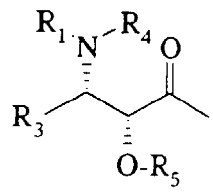

(V)

dans laquelle $R_1$ et $R_3$ sont définis comme précédemment, ou bien $R_4$ représente un atome d'hydrogène et $R_5$ représente un groupement protecteur de la fonction hydroxy, et ou bien $R_4$ et $R_5$ forment ensemble un hétérocycle, pour obtenir un produit de formule générale :

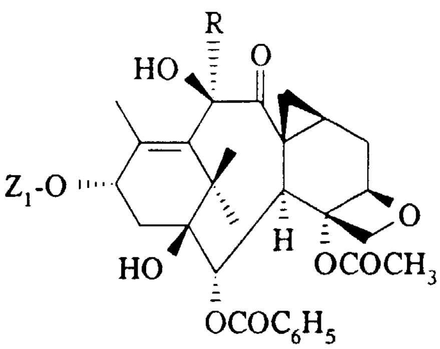

(VI)

suivi éventuellement du remplacement des groupements protecteurs représentés par $R_5$ et/ou $R_4$ et $R_5$ et portés par $Z_1$ par des atomes d'hydrogène.

Généralement, le procédé est mis en oeuvre dans un solvant organique inerte tel qu'un éther (tétrahydrofuranne) à une température comprise entre -78 et 30°C.

De préférence, $R_4$ représente un atome d'hydrogène et $R_5$ représente un groupement protecteur de la fonction hydroxy ou bien $R_4$ et $R_5$ forment ensemble un hétérocycle saturé à 5 ou 6 chaînons.

Lorsque $R_4$ représente un atome d'hydrogène, $R_5$ représente de préférence un radical méthoxyméthyle, éthoxy-1 éthyle, benzyloxyméthyle, triméthylsilyle, triéthylsilyle, β-triméthylsilyléthoxyméthyle, benzyloxycarbonyle ou tétrahydropyrannyle.

Lorsque $R_4$ et $R_5$ forment ensemble un hétérocycle, celui-ci est de préférence un cycle oxazolidine éventuellement mono-substitué ou gem-disubstitué en position -2.

Le remplacement des groupements protecteurs $R_5$ et/ou $R_4$ et $R_5$ par des atomes d'hydrogène peut être effectué, selon leur nature de la manière suivante :

1) lorsque $R_4$ représente un atome d'hydrogène et $R_5$ représente un groupement protecteur de la fonction hydroxy, le remplacement des groupements protecteurs par des atomes d'hydrogène s'effectue au moyen d'un acide minéral (acide chlorhydrique, acide sulfurique, acide fluorhydrique) ou organique (acide acétique, acide méthanesulfonique, acide trifluorométhanesulfonique, acide p.toluènesulfonique) utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,

2) lorsque $R_4$ et $R_5$ forment ensemble un hétérocycle saturé à 5 ou 6 chaînons et plus particulièrement un cycle oxazolidine de formule générale :

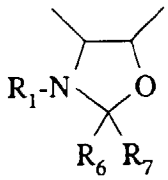

(VII)

dans laquelle $R_1$ est défini comme précédemment, $R_6$ et $R_7$, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien $R_6$ représente un radical alcoxy contenant 1 à 4 atomes de carbone ou un radical trihalométhyle tel que trichlorométhyle ou un radical phényle substitué par un radical trihalométhyle tel que trichlorométhyle et $R_7$ représente un atome d'hydrogène, ou bien $R_6$ et $R_7$ forment ensemble avec l'atome de carbone auquel ils sont liés un cycle ayant 4 à 7 chaînons, le remplacement du groupement protecteur formé par $R_6$ et $R_7$ par des atomes d'hydrogène peut être effectué, selon les significations de $R_1$, $R_6$ et $R_7$, de la manière suivante :

a) lorsque $R_1$ représente un radical tert-butoxycarbonyle, $R_6$ et $R_7$, identiques ou différents, représentent un radical alcoyle ou un radical aralcoyle (benzyle) ou aryle (phényle), ou bien $R_6$ représente un radical trihalométhyle ou un radical phényle substitué par un radical trihalométhyle, et $R_7$ représente un atome d'hydrogène, ou bien $R_6$ et $R_7$ forment ensemble un cycle ayant de 4 à 7 chaînons, le traitement de l'ester de formule générale (VI) par un acide minéral ou organique éventuellement dans un solvant organique tel qu'un alcool conduit au produit de formule générale :

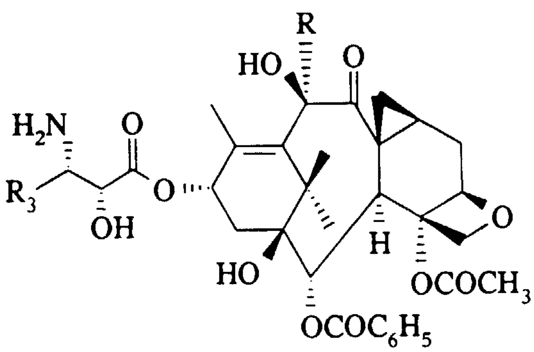

(VIII)

dans laquelle R et $R_3$ sont définis comme précédemment, qui est acylé au moyen de chlorure de benzoyle dans lequel le noyau phényle est éventuellement substitué, de chlorure de thénoyle, de chlorure de furoyle ou d'un produit de formule générale :

$$R_2\text{-O-CO-X} \qquad \text{(IX)}$$

dans laquelle $R_2$ est défini comme précédemment et X représente un atome d'halogène (fluor, chlore) ou un

reste -O-$R_2$ ou -O-CO-O-$R_2$, pour obtenir un produit de formule générale (I) dans laquelle Z représente un radical de formule générale (II).

De préférence, le produit de formule générale (VI) est traité par l'acide formique à une température voisine de 20°C.

De préférence, l'acylation du produit de formule générale (VIII) au moyen d'un chlorure de benzoyle dans lequel le radical phényle est éventuellement substitué, de chlorure de thénoyle, de chlorure de furoyle ou d'un produit de formule générale (IX) est effectuée dans un solvant organique inerte choisi parmi les esters tels que l'acétate d'éthyle, l'acétate d'isopropyle ou l'acétate de n.butyle et les hydrocarbures aliphatiques halogénés tels que le dichlorométhane ou le dichloro-1,2 éthane en présence d'une base minérale telle que le bicarbonate de sodium ou organique telle que la triéthylamine. La réaction est effectuée à une température comprise entre 0 et 50°C, de préférence voisine de 20°C.

b) lorsque $R_1$ représente un radical benzoyle éventuellement substitué, thénoyle ou furoyle ou un radical $R_2$O-CO- dans lequel $R_2$ est défini comme précédemment, $R_6$ représente un atome d'hydrogène ou un radical alcoxy contenant 1 à 4 atomes de carbone ou un radical phényle substitué par un ou plusieurs radicaux alcoxy contenant 1 à 4 atomes de carbone et $R_7$ représente un atome d'hydrogène, le remplacement du groupement protecteur formé par $R_6$ et $R_7$ par des atomes d'hydrogène s'effectue en présence d'un acide minéral (acide chlorhydrique, acide sulfurique) ou organique (acide acétique, acide méthanesulfonique, acide trifluorométhanesulfonique, acide p.toluènesulfonique) utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C, de préférence entre 15 et 30°C.

Le produit de formule générale (IV) peut être obtenu par oxydation d'un produit de formule générale :

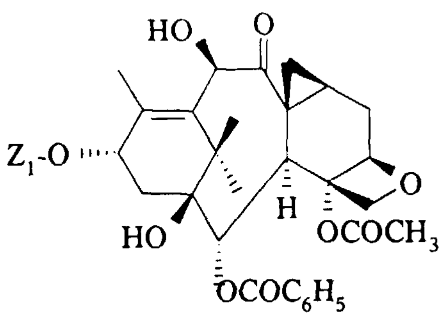

(X)

dans laquelle $Z_1$ est défini comme précédemment.

Généralement l'oxydation est réalisée au moyen d'un oxydant choisi, de préférence, parmi le chlorochromate de pyridinium, le dichromate de pyridinium, le bichromate de potassium, le bichromate d'ammonium, le bichromate de pyridinium, ou l'oxyde de manganèse dans des conditions qui ne touchent pas au reste de la molécule.

Selon la nature de l'oxydant utilisé, l'oxydation est mise en oeuvre en milieu organique anhydre ou en milieu hydro-organique.

Généralement, l'oxydation est réalisée à une température comprise entre 0 et 50°C.

Les produits de formule générale (X) peuvent être obtenus par action d'un halogénure de métal alcalin (chlorure de sodium, iodure de sodium, fluorure de potassium) ou d'un azoture de métal alcalin (azoture de sodium) ou d'un sel d'ammonium quaternaire ou d'un phosphate de métal alcalin sur un dérivé de la baccatine III ou de la désacétyl-10 baccatine III de formule générale :

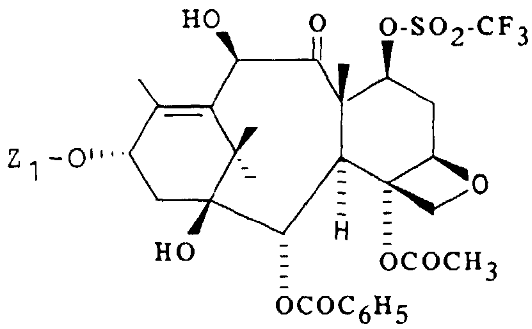

(XI)

dans laquelle $Z_1$ est défini comme précédemment.

Généralement la réaction est effectuée dans un solvant organique choisi parmi les éthers (tétrahydrofuranne, diisopropyléther, méthyl t.butyléther) et les nitriles (acétonitrile) seul ou en mélange à une température comprise entre 20°C et la température d'ébullition du mélange réactionnel.

Le produit de formule générale (XI) peut être obtenu par action par action d'un dérivé de l'acide trifluorométhanesulfonique tel que l'anhydride ou le N-phényl trifluorométhanesulfonimide sur un produit de formule générale :

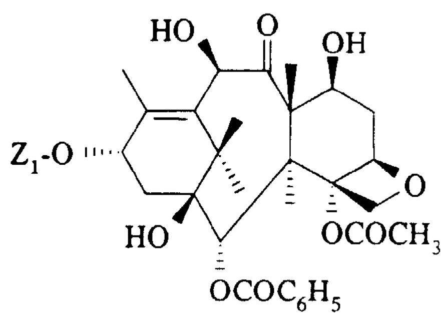

(XII)

dans laquelle $Z_1$ est défini comme précédemment.

Généralement, la réaction s'effectue dans un solvant organique inerte (hydrocarbures aliphatiques éventuellement halogénés, hydrocarbures aromatiques) en présence d'une base organique telle qu'une amine tertiaire aliphatique (triéthylamine) ou la pyridine à une température comprise entre -50 et +20°C.

Les produits de formule générale (XII) peuvent être préparés dans les conditions décrites dans les brevets européens EP 0 253 738 ou EP 0 336 841 ou dans la demande internationale PCT WO 92/09589.

Les nouveaux produits de formule générale (I) obtenus par la mise en oeuvre des procédés selon l'invention peuvent être purifiés selon les méthodes connues telles que la cristallisation ou la chromatographie.

Les produits de formule générale (I) dans laquelle Z représente un radical de formule générale (II) présentent des propriétés biologiques remarquables.

In vitro, la mesure de l'activité biologique est effectuée sur la tubuline extraite de cerveau de porc par la méthode de M.L. Shelanski et coll., Proc. Natl. Acad. Sci. USA, 70, 765-768 (1973). L'étude de la dépolymérisation des microtubules en tubuline est effectuée selon la méthode de G. Chauvière et coll., C.R. Acad. Sci., 293, série II, 501-503 (1981). Dans cette étude les produits de formule générale (I) dans laquelle Z représente un radical de formule générale (II) se sont montrés au moins aussi actifs que le taxol et le Taxotère.

In vivo, les produits de formule générale (I) dans laquelle Z représente un radical de formule générale (II) se sont montrés actifs chez la souris greffée par le mélanome B16 à des doses comprises entre 1 et 10 mg/kg par voie intrapéritonéale, ainsi que sur d'autres tumeurs liquides ou solides.

Les nouveaux produits ont des propriétés anti-tumorales et plus particulièrement une activité sur les tumeurs qui sont résistantes au Taxol® ou au Taxotère®. De telles tumeurs comprennent les tumeurs du colon qui ont une expression élevée du gène mdr 1 (gène de la multi-drug resistance). La multi-drug resistance est un terme habituel se rapportant à la résistance d'une tumeur à différents produits de structures et de mécanismes d'action différents. Les taxoïdes sont généralement connus pour être fortement reconnus par des tumeurs expérimentales telles que P388/DOX, une lignée cellulaire sélectionnée pour sa résistance à la doxorubicine (DOX) qui exprime mdr 1.

Les exemples suivants illustrent la présente invention.

EXEMPLE 1

A une solution de 260 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthylène-7,8β dioxo-9,10 nor-19 taxène-11 yle-13α dans 5 cm3 de tétrahydrofurane, maintenue sous atmosphère d'argon, à une température voisine de -78°C, on ajoute goutte à goutte 0,116cm3 d'une solution d'iodure de méthylmagnésium 3M dans l'éther éthylique. Après 75 minutes à -78°C, on ajoute goutte à goutte 0,116cm3 supplémentaire d'une solution d'iodure de méthylmagnésium 3M dans l'éther éthylique. Le mélange réactionnel est agité pendant 30 minutes à -78°C, puis pendant 18 heures à une température voisine de 0°C. Après refroidissement à une température voisine de -78°C, on ajoute goutte à goutte 0,116cm3 d'une solution d'iodure de méthylmagnésium 3M dans l'éther éthylique, puis laisse réagir pendant 20 minutes à -78°C, pendant 30 minutes à 0°C, puis pendant 30 minutes à 20°C. Le mélange réactionnel est traité par 1 cm3 d'une solution aqueuse saturée en chlorure d'ammonium et 5 cm3 d'acétate d'éthyle. Après décantation, la phase aqueuse est extraite par 1,5 cm3 d'acétate d'éthyle, les phases organiques sont rassemblées, séchées sur sulfate de magnésium, filtrées sur verre fritté et concentrées sous pression réduite (0,27 kPa) à une température voisine de 40°C. On obtient ainsi 240 mg d'une meringue orangée que l'on purifie par chromatographie à pression atmosphérique sur 10 g de silice (0,063-0,2 mm) contenus dans une colonne de 1,6 cm de diamètre (éluant : méthanol-dichlorométhane : 3-97 en volumes) en recueillant des fractions de 5 cm3. Les fractions ne contenant que le produit cherché sont réunies et concentrées à sec sous pression réduite (0,27 kPa) à 40°C pendant 2heures. On obtient ainsi 0,15 g de tert-butoxycarbonyl-3 (méthoxy-4 phényl)-2 phényl4 oxazolidine-1,3 carboxylate-5 (2R,4S,5R) d'acétoxy-4α benzoyloxy-2α époxy-5β,20 dihydroxy-1β,10β méthyl-10α méthylène-7,8β oxo-9 nor-19 taxène-11 yle-13α sous forme d'une meringue jaune vif.

Une solution de 75 mg de tert-butoxycarbonyl-3 (méthoxy-4 phényl)-2 phényl-4 oxazolidine-1,3 carboxylate-5 (2R, 4S,5R) d'acétoxy-4α benzoyloxy-2α époxy-5β,20 dihydroxy-1β,10β méthyl-10α méthylène-7,8β oxo-9 nor-19 taxène-11 yle-13α dans 1,5 cm3 d'une solution d'acide chlorhydrique 0,1N dans l'éthanol est agitée, à une température voisine de 20°C, pendant 5 heures. On ajoute 0,5 cm3 supplémentaire d'acide chlorhydrique 0,1N dans l'éthanol. Après 16 heures à une température voisine de 5°C, on ajoute 0,5 cm3 supplémentaire d'acide chlorhydrique 0,1N dans l'éthanol et la solution est agitée pendant encore 3 heures à une température voisine de 20°C. Le mélange réactionnel est dilué par 2,5 cm3 de dichlorométhane, 2,5 cm3 d'une solution aqueuse saturée en hydrogénocarbonate de sodium et 2 cm3 d'eau distillée. Après décantation, la phase aqueuse est extraite par 5 cm3 de dichlorométhane, les phases organiques sont rassemblées, séchées sur sulfate de magnésium, filtrées sur verre fritté et concentrées sous pression réduite (0,27 kPa) à une température voisine de 40°C. On obtient ainsi 124 mg d'une meringue jaune que l'on purifie par chromatographie préparative sur couche mince [5 plaques préparatives Merck, Kieselgel 60F254, épaisseur 0,5 mm, dépôt en solution dans le dichlorométhane, éluant : mélange méthanol-dichlorométhane (5-95 en volumes)]. Après élution de la zone correspondant au produit principal par un mélange méthanol-dichlorométhane (10-90 en volumes), filtration sur verre fritté, puis évaporation des solvants sous pression réduite (0,27 kPa) à une température voisine de 40°C, on obtient 37 mg de tert-butoxycarbonylamino-3 hydroxy-2 phényl-3 propionate-(2R,3S) d'acétoxy-4α benzoyloxy-2α époxy-5β,20 dihydroxy-1β,10β méthyl-10α méthylène-7,8β oxo-9 nor-19 taxène-11 yle-13α sous forme d'une meringue blanche dont les caractéristiques sont les suivantes :

- spectre de R.M.N. $^1H$ (600 MHz, $CDCl_3$, δ en ppm) : 1,21 (s, 3H : $CH_3$) ; 1,22 (s, 9H : $C(CH_3)_3$); 1,28 (s, 3H : $CH_3$) ; 1,38 (mt, 1H : H en 7) ; 1,68 et 2,31 (2 mt, 1H chacun : $CH_2$ en 19) ; 1,70 (s, 3H : $CH_3$ en 10) ; 1,85 (s, 1H : 0H en 1) ; 1,87 (s, 3H: $CH_3$) ; 2,17 et 2,33 (2 mt, 1H chacun : $CH_2$ en 14) ; 2,17 et 2,43 (respectivement d et dt, J = 16 et J = 16 et 4,5 Hz, 1H chacun : $CH_2$ en 6) ; 2,41 (s, 3H : $COCH_3$) ; 3,25 (mt, 1H : OH en 2') ; 4,05 et 4,35 (2d, J = 9 Hz, 1H chacun : $CH_2$ en 20) ; 4,30 (d, J = 7 Hz, 1H : H en 3); 4,42 (s, 1H : OH en 10) ; 4,60 (mt, 1H : H en 2') ; 4,75 (d, J = 4 Hz, 1H : H en 5) ; 5,30 (mt, 1H : H en 3') ; 5,38 (d, J =10 Hz, 1H : CONH) ; 5,67 (d, J = 7 Hz, 1H : H en 2) ; 6,33 (t large, J = 9 Hz, 1H : H en 13) 7,30 (t, J = 7,5 Hz, 1H : aromatique en 3' H en para) ; 7,37 (d, J = 7,5 Hz, 2H : aromatique en 3' H en ortho) ;7,40 (t, J = 7,5 Hz, 2H : aromatique en 3' H en méta) ; 7,51 (t, J = 7,5 Hz, 2H : $OCOC_6H_5$ H en méta) ; 7,60 (t, J = 7,5 Hz, 1H : $OCOC_6H_5$ H en para) ; 8,17 (d, J = 7,5 Hz, 2H : $OCOC_6H_5$ H en ortho).

Le tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthylène-7,8β dioxo-9,10 nor-19 taxène-11 yle-13α peut être préparé de la manière suivante :

A une solution de 900 mg de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β méthylène-7,8β oxo-9 nor-19 taxène-11 yle-13α dans 9 cm3 de dichlorométhane, maintenue sous atmosphère d'argon, à une température voisine de 20°C, on ajoute 0,5 g de tamis moléculaire 4Å, puis par portions 0,43 g de chlorochromate de pyridinium. Le mélange réactionnel est agité 90 minutes à une température voisine de 20°C, puis filtré sur verre fritté garni de Clarcel. Après rinçage du

résidu solide par du dichlorométhane, les filtrats sont concentrés sous pression réduite (0,27 kPa) à une température voisine de 40°C. On obtient ainsi 1,13 g d'une laque brune que l'on purifie par chromatographie à pression atmosphérique sur 40 g de silice (0,063-0,2 mm) contenus dans une colonne de 2,2 cm de diamètre (éluant : méthanol-dichlorométhane 2-98 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 0,63 g de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 hydroxy-1β méthylène-7,8β dioxo-9,10 nor-19 taxène-11 yle-13α sous forme d'une meringue jaune-vert.

Le tert-butoxycarbonyl-3 (méthoxy-4 phényl)-2 phényl4 oxazolidine-1,3 carboxylate-5-(2R,4S,5R) d'acétoxy-4α benzoyloxy-2α époxy-5β,20 dihydroxy1β,10β méthylène-7,8β oxo-9 nor-19 taxène-11 yle-13α peut être préparé de la manière suivante :

A une solution de 1,8 g de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β oxo-9 trifluorométhanesulfonyloxy-7β taxène-11 yle13α dans 20 cm3 d'acétonitrile et 3 cm3 de tétrahydrofurane on ajoute 1,5 g de chlorure de sodium et 0,5 g de tamis moléculaire 4Å. Le mélange réactionnel est porté au reflux, sous atmosphère inerte d'argon, pendant 1 heure, ramené à une température voisine de 20°C, et filtré sur verre fritté. Le résidu solide est rincé par 10 cm3 d'acétate d'éthyle. Les filtrats sont rassemblés, séchés sur sulfate de magnésium, filtrés sur verre fritté et concentrés sous pression réduite (0,27 kPa) à une température voisine de 40°C. On obtient ainsi 1,6 g d'une meringue jaune que l'on purifie par chromatographie à pression atmosphérique sur 60 g de silice (0,063-0,2 mm) contenus dans une colonne de 3 cm de diamètre (éluant : acétate d'éthyledichlorométhane : 20-80 en volumes) en recueillant des fractions de 10 cm3. Les fractions ne contenant que le produit cherché sont réunies et concentrées à sec sous pression réduite (0,27 kPa) à 40°C pendant 2 heures. On obtient ainsi 0,91 g de tertbutoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 dihydroxy-1β,10β méthylène7,8β oxo-9 nor-19 taxène-11 yle-13α sous forme d'une meringue blanche.

Le tert-butoxycarbonyl-3 (méthoxy-4 phényl)-2 phényl4 oxazolidine-1,3 carboxylate-5 (2R,4S,5R) d'acétoxy-4α benzoyloxy-2α époxy-5β,20 dihydroxy1β,10β oxo-9 trifluorométhanesulfonate-7β taxène-11 yle-13α peut être préparé de la manière suivante :

A une solution de 1,9 g de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 trihydroxy-1β,7β,10β oxo-9 taxène-11 yle-13α dans 20 cm3 de dichlorométhane anhydre et 0,68 cm3 de pyridine anhydre, maintenue sous atmosphère d'argon, à une température voisine de -35°C, on ajoute goutte à goutte 0,54 cm3 d'anhydride trifluorométhanesulfonique. Le mélange réactionnel est agité 10 minutes à -35°C, 90 minutes à une température voisine de -5°C, puis additionné à une température voisine de -15°C de 5 cm3 d'eau distillée. Après décantation, la phase aqueuse est réextraite par 2 cm3 de dichlorométhane, les phases organiques sont rassemblées, séchées sur sulfate de magnésium, filtrées sur verre fritté et concentrées sous pression réduite (0,27 kPa) à une température voisine de 40°C. On obtient ainsi 2,4 g d'une huile jaune que l'on purifie par chromatographie à pression atmosphérique sur 100 g de silice (0,063-0,2 mm) contenus dans une colonne de 3,2 cm de diamètre (éluant : méthanol-dichlorométhane : 2-98 en volumes) en recueillant des fractions de 15 cm3. Les fractions ne contenant que le produit cherché sont réunies et concentrées à sec sous pression réduite (0,27 kPa) à 40°C pendant 2 heures. On obtient ainsi 1,23 g de tert-butoxycarbonyl-3 (méthoxy-4 phényl)-2 phényl-4 oxazolidine-1,3 carboxylate-5 (2R,4S,5R) d'acétoxy-4α benzoyloxy-2α époxy-5β,20 dihydroxy1β,10β oxo-9 trifluorométhanesulfonate-7β taxène-11 yle-13α sous forme d'une meringue jaune pâle.

Le tert-butoxycarbonyl-3 (méthoxy-4 phényl)-2 phényl-4 oxazolidine-1,3 carboxylate-5-(2R,4S,5R)) d'acétoxy-4α benzoyloxy-2α époxy-5β,20 trihydroxy1,7β,10β oxo-9 taxène-11 yle-13α peut être préparé de la manière suivante :

Une solution de 3,95 g de tert-butoxycarbonyl-3 (méthoxy-4 phényl)-2 phényl-4 oxazolidine-1,3 carboxylate-5-(2R,4S,5R) d'acétoxy-4 benzoyloxy-2α époxy-5β,20 hydroxy-1β oxo-9 bis-(trichloro-2,2,2 éthoxy)carbonyloxy-7β,10β taxène-11 yle-13α dans un mélange de 20 cm3 de méthanol et de 7 cm3 d'acide acétique est chauffée sous agitation, sous atmosphère d'argon, jusqu'à une température voisine de 60°C, puis additionnée de 2 g de zinc en poudre. Le mélange réactionnel est ensuite agité pendant 15 minutes à 60°C puis refroidi à une température voisine de 20°C et filtré sur verre fritté garni de célite. Le verre fritté est lavé par 2 fois 15 cm3 de méthanol. Le filtrat est concentré à sec sous pression réduite (0,27 kPa) à une température voisine de 40°C. Le résidu est additionné de 100 cm3 de dichlorométhane. La phase organique est lavée par 2 fois 10 cm3 d'une solution aqueuse saturée d'hydrogénocarbonate de sodium puis par 10 cm3 d'eau distillée. La phase organique est séchée sur sulfate de magnésium, filtrée sur verre fritté, puis concentrée à sec sous pression réduite (2,7 kPa) à 40°C. On obtient 5,57 g d'une meringue blanche que l'on purifie par chromatographie sur 300 g de silice (0,063-0,2 mm) contenus dans une colonne de 6 cm de diamètre (gradient d'élution : méthanol-dichlorométhane : de 0-100 à 2-98 en volumes) en recueillant des fractions de 100cm3. Les fractions ne contenant que le produit cherché sont réunies et concentrées à sec sous pression réduite (0,27 kPa) à 40°C pendant 2 heures. On obtient ainsi 2,52 g de tert-butoxycarbonyl-3 (méthoxy-4 phényl)-2 phényl-4 oxazolidinecarboxylate-5-(2R,4S,5R)) d'acétoxy-4 benzoyloxy-2α époxy-5β,20 trihydroxy-1,7β,10β oxo-9 taxène-11 yle-13α

sous forme d'une meringue blanche.

Le tert-butoxycarbonyl-3 (méthoxy-4 phényl)-2 phényl-4 oxazolidine-1,3 carboxylate-5-(2R,4S,5R)) d'acétoxy-4 benzoyloxy-2α époxy-5β,20 hydroxy-1β oxo-9 bis-(trichloro-2,2,2 éthoxy)carbonyloxy-7β,10β taxène-11 yle-13α peut être préparé dans les conditions décrites dans la demande internationale PCT WO 94/07878.

Les nouveaux produits de formule générale (I) dans laquelle Z représente un radical de formule générale (II) manifestent une activité inhibitrice significative de la prolifération cellulaire anormale et possèdent des propriétés thérapeutiques permettant le traitement de malades ayant des conditions pathologiques associées à une prolifération cellulaire anormale. Les conditions pathologiques incluent la prolifération cellulaire anormale de cellules malignes ou non malignes de divers tissus et/ou organes, comprenant, de manière non limitative, les tissus musculaires, osseux ou conjonctifs, la peau, le cerveau, les poumons, les organes sexuels, les systèmes lymphatiques ou rénaux, les cellules mammaires ou sanguines, le foie, l'appareil digestif, le pancréas et les glandes thyroïdes ou adrénales. Ces conditions pathologiques peuvent inclure également le psoriasis, les tumeurs solides, les cancers de l'ovaire, du sein, du cerveau, de la prostate, du colon, de l'estomac, du rein ou des testicules, le sarcome de Kaposi, le cholangiocarcinome, le choriocarcinome, le neuroblastome, la tumeur de Wilms, la maladie de Hodgkin, les mélanomes, les myélomes multiples, les leucémies lymphocytaires chroniques, les lymphomes granulocytaires aigus ou chroniques. Les nouveaux produits selon l'invention sont particulièrement utiles pour le traitement du cancer de l'ovaire. Les produits selon l'invention peuvent être utilisés pour prévenir ou retarder l'apparition ou la réapparition des conditions pathologiques ou pour traiter ces conditions pathologiques.

Les produits selon l'invention peuvent être administrés à un malade selon différentes formes adaptées à la voie d'administration choisie qui, de préférence, est la voie parentérale. L'administration par voie parentérale comprend les administrations intraveineuse, intrapéritonéale, intramusculaire ou sous-cutanée. Plus particulièrement préférée est l'administration intrapéritonéale ou intraveineuse.

La présente invention comprend également les compositions pharmaceutiques qui contiennent au moins un produit de formule générale (I) en une quantité suffisante adaptée à l'emploi en thérapeutique humaine ou vétérinaire. Les compositions peuvent être préparées selon les méthodes habituelles en utilisant un ou plusieurs adjuvants, supports ou excipients pharmaceutiquement acceptables. Les supports convenables incluent les diluants, les milieux aqueux stériles et divers solvants non toxiques. De préférence les compositions se présentent sous forme de solutions ou de suspensions aqueuses, de solutions injectables qui peuvent contenir des agents émulsifiants, des colorants, des préservatifs ou des stabilisants.

Le choix des adjuvants ou excipients peut être déterminé par la solubilité et les propriétés chimiques du produit, le mode particulier d'administration et les bonnes pratiques pharmaceutiques.

Pour l'administration parentérale, on utilise des solutions ou des suspensions stériles aqueuses ou non aqueuses. Pour la préparation de solutions ou de suspensions non aqueuses peuvent être utilisés des huiles végétales naturelles telle que l'huile d'olive, l'huile de sésame ou l'huile de paraffine ou les esters organiques injectables tel que l'oléate d'éthyle. Les solutions stériles aqueuses peuvent être constituées d'une solution d'un sel pharmaceutiquement acceptable en solution dans de l'eau. les solutions aqueuses conviennent pour l'administration intraveineuse dans la mesure où le pH est convenablement ajusté et où l'isotonicité est réalisée, par exemple, par une quantité suffisante de chlorure de sodium ou de glucose. La stérilisation peut être réalisée par chauffage ou par tout autre moyen qui n'altère pas la composition.

Il est bien entendu que tous les produits entrant dans les compositions selon l'invention doivent être purs et non toxiques pour les quantités utilisées.

Les compositions peuvent contenir au moins 0,01 % de produit thérapeutiquement actif. La quantité de produit actif dans une composition est telle qu'une posologie convenable puisse être prescrite. De préférence, les compositions sont préparées de telle façon qu'une dose unitaire contienne de 0,01 à 1000 mg environ de produit actif pour l'administration par voie parentérale.

Le traitement thérapeutique peut être effectué concuremment avec d'autres traitements thérapeutiques incluant des médicaments antinéoplastiques, des anticorps monoclonaux, des thérapies immunologiques ou des radiothérapies ou des modificateurs des réponses biologiques. Les modificateurs des réponses incluent, de manière non limitative, les lymphokines et les cytokines telles que les interleukines, les interférons (α, β ou δ) et le TNF. D'autres agents chimiothérapeutiques utiles dans le traitement des désordres dus à la prolifération anormale des cellules incluent, de manière non limitative, les agents alkylants tels que les moutardes à l'azote comme la mechloretamine, le cyclophosphamide, le melphalan et le chlorambucil, des sulfonates d'alkyle comme le busulfan, les nitrosourées comme la carmustine, la lomustine, la sémustine et la streptozocine, les triazènes comme la dacarbazine, les antimétabolites comme les analogues de l'acide folique tel que le méthotrexate, les analogues de pyrimidine comme le fluorouracil et la cytarabine, des analogues de purines comme la mercaptopurine et la thioguanine, des produits naturels tels que les alcaloïdes de vinca comme la vinblastine, la vincristine et la vendésine, des épipodophyllotoxines comme l'étoposide et le teniposide, des antibiotiques comme la dactinomycine, la daunorubicine, la doxorubicine, la bléomycine, la plicamycine et la mitomycine, des enzymes comme la L-asparaginase, des agents divers comme les complexes de coordination

du platine tel que le cisplatine, les urées substituées tel que l'hydroxyurée, les dérivés de méthylhydrazine comme la procarbazine, les suppresseurs adrénocorticoïques comme le mitotane et l'aminoglutéthymide, les hormones et les antagonistes comme les adrénocorticostéroïdes comme la prednisone, les progestines comme le caproate d'hydroxyprogestérone, l'acétate de méthoxyprogestérone et l'acétate de megestrol, les oestrogènes comme le diéthylstilbestrol et l'éthynylestradiol, les antioestrogènes comme le tamoxifène, les androgènes comme le propionate de testostérone et la fluoxymesterone.

Les doses utilisées pour mettre en oeuvre les méthodes selon l'invention sont celles qui permettent un traitement prophylactique ou un maximum de réponse thérapeutique. Les doses varient selon la forme d'administration, le produit particulier sélectionné et les caractéristiques propres du sujet à traiter. En général, les doses sont celles qui sont thérapeutiquement efficaces pour le traitement des désordres dus à une prolifération cellulaire anormale. Les produits selon l'invention peuvent être administrés aussi souvent que nécessaire pour obtenir l'effet thérapeutique désiré. Certains malades peuvent répondre rapidement à des doses relativement fortes ou faibles puis avoir besoin de doses d'entretien faibles ou nulles. Généralement, de faibles doses seront utilisées au début du traitement et, si nécessaire, des doses de plus en plus fortes seront administrées jusqu'à l'obtention d'un effet optimum. Pour d'autres malades il peut être nécessaire administrer des doses d'entretien 1 à 8 fois par jour, de préférence 1 à 4 fois, selon les besoins physiologiques du malade considéré. Il est aussi possible que pour certains malades il soit nécessaire de n'utiliser qu'une à deux administrations journalières.

Chez l'homme, les doses sont généralement comprises entre 0,01 et 200 mg/kg. Par voie intrapéritonéale, les doses seront en général comprises entre 0,1 et 100 mg/kg et, de préférence entre 0,5 et 50 mg/kg et , encore plus spécifiquement entre 1 et 10 mg/kg. Par voie intraveineuse, les doses sont généralement comprises entre 0,1 et 50 mg/kg et, de préférence entre 0,1 et 5 mg/kg et, encore plus spécifiquement entre 1 et 2 mg/kg. Il est entendu que, pour choisir le dosage le plus approprié, devront être pris en compte la voie d'administration, le poids du malade, son état de santé général, son âge et tous les facteurs qui peuvent influer sur l'efficacité du traitement.

L'exemple suivant illustre une composition selon l'invention.

EXEMPLE

On dissout 40 mg du produit obtenu à l'exemple 1 dans 1 cm3 d'Emulphor EL 620 et 1 cm3 d'éthanol puis la solution est diluée par addition de 18 cm3 de sérum physiologique.

La composition est administrée par perfusion pendant 1 heure par introduction dans du soluté physiologique.

**Revendications**

**1.** Nouveaux taxoïdes de formule générale :

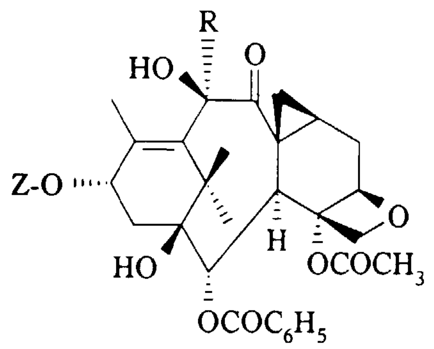

(I)

dans laquelle :

- R représente un radical alcoyle droit ou ramifié contenant 1 à 6 atomes de carbone, alcényle droit ou ramifié contenant 2 à 6 atomes de carbone, alcynyle droit ou ramifié contenant 2 à 6 atomes de carbone, cycloalcoyle contenant 3 à 6 atomes de carbone, cycloalcényle contenant 4 à 6 atomes de carbone, aryle, hétérocyclyle aromatique contenant 5 à 6 chaînons

Z représente un atome d'hydrogène ou un radical de formule générale :

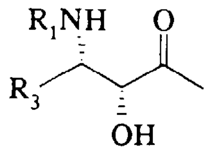

(II)

dans laquelle :

$R_1$ représente un radical benzoyle éventuellement substitué par un ou plusieurs atomes ou radicaux, identiques ou différents, choisis parmi les atomes d'halogène et les radicaux alcoyles contenant 1 à 4 atomes de carbone, alcoxy contenant 1 à 4 atomes de carbone ou trifluorométhyle, thénoyle ou furoyle ou un radical $R_2$-O-CO- dans lequel $R_2$ représente :

- un radical alcoyle contenant 1 à 8 atomes de carbone, alcényle contenant 2 à 8 atomes de carbone, alcynyle contenant 3 à 8 atomes de carbone, cycloalcoyle contenant 3 à 6 atomes de carbone, cycloalcényle contenant 4 à 6 atomes de carbone, bicycloalcoyle contenant 7 à 10 atomes de carbone, ces radicaux étant éventuellement substitués par un ou plusieurs substituants choisis parmi les atomes d'halogène et les radicaux hydroxy, alcoxy contenant 1 à 4 atomes de carbone, dialcoylamino dont chaque partie alcoyle contient 1 à 4 atomes de carbone, pipéridino, morpholino, pipérazinyl-1 (éventuellement substitué en -4 par un radical alcoyle contenant 1 à 4 atomes de carbone ou par un radical phénylalcoyle dont la partie alcoyle contient 1 à 4 atomes de carbone), cycloalcoyle contenant 3 à 6 atomes de carbone, cycloalcényle contenant 4 à 6 atomes de carbone, phényle (éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone), cyano, carboxy ou alcoxycarbonyle dont la partie alcoyle contient 1 à 4 atomes de carbone,
- un radical phényle ou α- ou β-naphtyle éventuellement substitué par un ou plusieurs atomes ou radicaux choisis parmi les atomes d'halogène et les radicaux alcoyles contenant 1 à 4 atomes de carbone ou alcoxy contenant 1 à 4 atomes de carbone ou un radical hétérocyclique aromatique à 5 chaînons choisi de préférence parmi les radicaux furyle et thiényle,
- ou un radical hétérocyclyle saturé contenant 4 à 6 atomes de carbone éventuellement substitué par un ou plusieurs radicaux alcoyles contenant 1 à 4 atomes de carbone,

$R_3$ représente un radical alcoyle droit ou ramifié contenant 1 à 8 atomes de carbone, alcényle droit ou ramifié contenant 2 à 8 atomes de carbone, alcynyle droit ou ramifié contenant 2 à 8 atomes de carbone, cycloalcoyle contenant 3 à 6 atomes de carbone, phényle ou α- ou β-naphtyle éventuellement substitué par un ou plusieurs atomes ou radicaux choisis parmi les atomes d'halogène et les radicaux alcoyles, alcényles, alcynyles, aryles, aralcoyles, alcoxy, alcoylthio, aryloxy, arylthio, hydroxy, hydroxyalcoyle, mercapto, formyle, acyle, acylamino, aroylamino, alcoxycarbonylamino, amino, alcoylamino, dialcoylamino, carboxy, alcoxycarbonyle, carbamoyle, alcoylcarbamoyle, dialcoylcarbamoyle, cyano, nitro et trifluorométhyle, ou un hétérocycle aromatique ayant 5 chaînons et contenant un ou plusieurs hétéroatomes, identiques ou différents, choisis parmi les atomes d'azote, d'oxygène ou de soufre et éventuellement substitué par un ou plusieurs substituants, identiques ou différents, choisis parmi les atomes d'halogène et les radicaux alcoyles, aryles, amino, alcoylamino, dialcoylamino, alcoxycarbonylamino, acyle, arylcarbonyle, cyano, carboxy, carbamoyle, alcoylcarbamoyle, dialcoylcarbamoyle ou alcoxycarbonyle, étant entendu que, dans les substituants des radicaux phényle, α- ou β-naphtyle et hétérocyclyles aromatiques, les radicaux alcoyles et les portions alcoyles des autres radicaux contiennent 1 à 4 atomes de carbone et que les radicaux alcényles et alcynyles contiennent 2 à 8 atomes de carbone et que les radicaux aryles sont des radicaux phényles ou α- ou β-naphtyles

**2.** Nouveaux taxoïdes selon la revendication 1 pour lesquels R représente un radical alcoyle contenant 1 à 4 atomes de carbone, Z représente un atome d'hydrogène ou un radical de formule générale (II) dans laquelle $R_1$ représente un radical benzoyle ou un radical $R_2$-O-CO- dans lequel $R_2$ représente un radical tert-butyle et $R_3$ représente un radical alcoyle contenant 1 à 6 atomes de carbone, alcényle contenant 2 à 6 atomes de carbone, cycloalcoyle contenant 3 à 6 atomes de carbone, phényle éventuellement substitué par un ou plusieurs atomes ou radicaux, identiques ou différents choisis parmi les atomes d'halogène (fluor, chlore) et les radicaux alcoyles, alcoxy, dialcoylamino, acylamino, alcoxycarbonylamino ou trifluorométhyle ou un radical furyle-2 ou -3, thiényle-2 ou -3 ou thiazolyle-2, -4 ou -5.

3. Nouveaux taxoïdes selon la revendication 1 pour lesquels R représente un radical méthyle, Z représente un atome d'hydrogène ou un radical de formule générale (II) dans laquelle $R_1$ représente un radical benzoyle ou un radical $R_2$-O-CO- dans lequel $R_2$ représente un radical tert-butyle et $R_3$ représente un radical isobutyle, isobuténylе, buténylе, cyclohexyle, phényle, furyle-2, furyle-3, thiényle-2, thiényle-3, thiazolyle-2, thiazolyle-4 ou thiazolyle-5.

4. Procédé de préparation d'un nouveau taxoide selon l'une des revendications 1 à 3 caractérisé en ce que l'on fait réagir un dérivé organométallique de formule générale :

R-X (III)

dans laquelle R est défini comme précédemment et X représente un atome de métal tel qu'un atome de lithium ou un reste d'organo-magnésien sur un produit de formule générale :

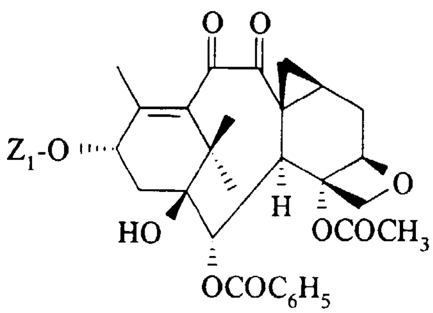

(IV)

dans laquelle $Z_1$ représente un atome d'hydrogène ou un radical de formule générale :

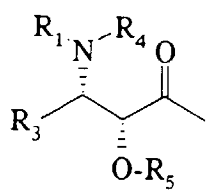

(V)

dans laquelle dans laquelle $R_1$ et $R_3$ sont définis comme dans l'une des revendications 1 à 3, ou bien $R_4$ représente un atome d'hydrogène et $R_5$ représente un groupement protecteur de la fonction hydroxy, et ou bien $R_4$ et $R_5$ forment ensemble un hétérocycle, pour obtenir un produit de formule générale :

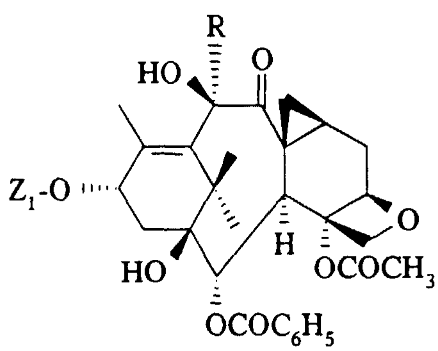

(VI)

puis remplace éventuellement les groupements protecteurs représentés par $R_5$ et/ou $R_4$ et $R_5$ par des atomes d'hydrogène.

5. Procédé selon la revendication 4 caractérisé en ce que l'on fait réagir le dérivé métallique en opérant dans un

solvant organique inerte à une température comprise entre -78 et +30°C.

6. Procédé selon la revendication 4 caractérisé en ce que le remplacement des groupements protecteurs $R_5$ et/ou $R_4$ et $R_5$ est effectué de la manière suivante :

1) lorsque $R_4$ représente un atome d'hydrogène et $R_5$ représente un groupement protecteur de la fonction hydroxy, le remplacement des groupements protecteurs par des atomes d'hydrogène s'effectue au moyen d'un acide minéral ou organique utilisé seul ou en mélange en opérant dans un solvant organique choisi parmi les alcools, les éthers, les esters, les hydrocarbures aliphatiques, les hydrocarbures aliphatiques halogénés, les hydrocarbures aromatiques ou les nitriles à une température comprise entre -10 et 60°C,
2) lorsque $R_4$ et $R_5$ forment ensemble un cycle oxazolidine de formule générale :

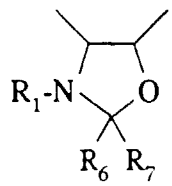

(VII)

dans laquelle $R_1$ est défini comme dans l'une des revendications 1 à 3, $R_6$ et $R_7$, identiques ou différents, représentent un atome d'hydrogène ou un radical alcoyle contenant 1 à 4 atomes de carbone, ou un radical aralcoyle dont la partie alcoyle contient 1 à 4 atomes de carbone et la partie aryle représente, de préférence, un radical phényle éventuellement substitué par un ou plusieurs radicaux alcoxy contenant 1 à 4 atomes de carbone, ou un radical aryle représentant, de préférence un radical phényle éventuellement substitué par un ou plusieurs radicaux alcoxy contenant 1 à 4 atomes de carbone, ou bien $R_6$ représente un radical alcoxy contenant 1 à 4 atomes de carbone ou un radical trihalométhyle tel que trichlorométhyle ou un radical phényle substitué par un radical trihalométhyle tel que trichlorométhyle et $R_7$ représente un atome d'hydrogène, ou bien $R_6$ et $R_7$ forment ensemble avec l'atome de carbone auquel ils sont liés un cycle ayant 4 à 7 chaînons, le remplacement du groupement protecteur formé par $R_6$ et $R_7$ par des atomes d'hydrogène peut être effectué, selon les significations de $R_1$, $R_6$ et $R_7$, de la manière suivante :

a) lorsque $R_1$ représente un radical tert-butoxycarbonyle, $R_6$ et $R_7$, identiques ou différents, représentent un radical alcoyle ou un radical aralcoyle ou aryle, ou bien $R_6$ représente un radical trihalométhyle ou un radical phényle substitué par un radical trihalométhyle, et $R_7$ représente un atome d'hydrogène, ou bien $R_6$ et $R_7$ forment ensemble un cycle ayant de 4 à 7 chaînons, le traitement de l'ester de formule générale (VI) par un acide minéral ou organique éventuellement dans un solvant organique tel qu'un alcool conduit au produit de formule générale :

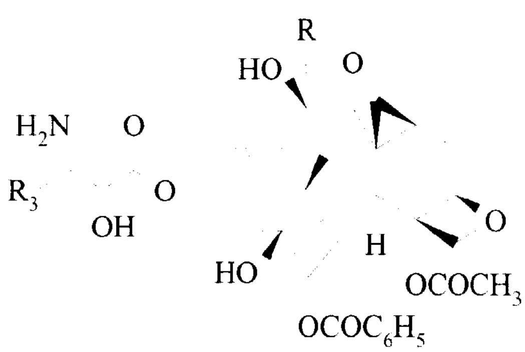

(VIII)

dans laquelle R et $R_3$ sont définis comme dans l'une des revendications 1 à 3, qui est acylé au moyen de chlorure de benzoyle dans lequel le noyau phényle est éventuellement substitué, de chlorure de thénoyle, de chlorure de furoyle ou d'un produit de formule générale :

$R_2$-O-CO-X (IX)

dans laquelle $R_2$ est défini comme précédemment et X représente un atome d'halogène (fluor, chlore) ou un reste -O-$R_2$ ou -O-CO-O-$R_2$, pour obtenir un produit de formule générale (I) dans laquelle Z représente un radical de formule générale (II),

b) lorsque $R_1$ représente un radical benzoyle éventuellement substitué, thénoyle ou furoyle ou un radical $R_2$O-CO- dans lequel $R_2$ est défini comme dans l'une des revendications 1 à 3, $R_6$ représente un atome d'hydrogène ou un radical alcoxy contenant 1 à 4 atomes de carbone ou un radical phényle substitué par un ou plusieurs radicaux alcoxy contenant 1 à 4 atomes de carbone et $R_7$ représente un atome d'hydrogène, le remplacement du groupement protecteur formé par $R_6$ et $R_7$ par des atomes d'hydrogène s'effectue en présence d'un acide minéral ou organique utilisé seul ou en mélange en quantité stoechiométrique ou catalytique, en opérant dans un solvant organique choisi parmi les alcools, les éthers, les esters, les hydrocarbures aliphatiques, les hydrocarbures aliphatiques halogénés et les hydrocarbures aromatiques à une température comprise entre -10 et 60°C.

**7.** Composition pharmaceutique caractérisée en ce qu'elle contient au moins un produit selon l'une des revendications 1 à 3 pour lequel Z représente un radical de formule générale (II) en association avec un ou plusieurs produits pharmaceutiquement acceptables qu'ils soient inertes ou pharmacologiquement actifs.

**Patentansprüche**

**1.** Neue Taxoide der allgemeinen Formel:

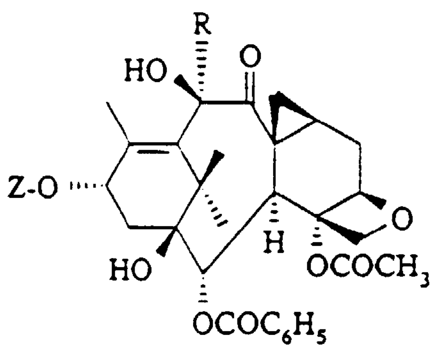

(I)

in der:

- R einen geraden oder verzweigten Alkylrest mit 1 bis 6 Kohlenstoffatomen, einen geraden oder verzweigten Alkenylrest mit 2 bis 6 Kohlenstoffatomen, einen geraden oder verzweigten Alkinylrest mit 2 bis 6 Kohlenstoffatomen, einen Cycloalkylrest mit 3 bis 6 Kohlenstoffatomen, einen Cycloalkenylrest mit 4 bis 6 Kohlenstoffatomen, einen Arylrest, einen aromatischen heterocyclischen Rest mit 5 bis 6 Kettengliedern darstellt,

Z ein Wasserstoffatom oder einen Rest der allgemeinen Formel:

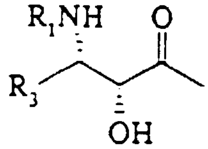

(II)

darstellt, in der:

$R_1$ einen Benzoylrest darstellt, der gegebenenfalls mit einem oder mehreren, gleichen oder verschiedenen Atomen oder Resten substituiert ist, die ausgewählt sind unter den Halogenatomen und den Alkylresten mit 1 bis 4 Kohlenstoffatomen, den Alkoxyresten mit 1 bis 4 Kohlenstoffatomen oder dem Trifluormethyl-, Thenoyl- oder Furoylrest oder einem Rest $R_2$-O-CO-, in dem $R_2$ darstellt:

- einen Alkylrest mit 1 bis 8 Kohlenstoffatomen, einen Alkenylrest mit 2 bis 8 Kohlenstoffatomen, einen Alkinylrest mit 3 bis 8 Kohlenstoffatomen, einen Cycloalkylrest mit 3 bis 6 Kohlenstoffatomen, einen Cycloalkenylrest mit 4 bis 6 Kohlenstoffatomen, einen Bicycloalkylrest mit 7 bis 10 Kohlenstoffatomen, wobei diese Reste gegebenenfalls mit einem oder mehreren Substituenten substituiert sind, die unter den Halogenatomen und dem Hydroxyrest, den Alkoxyresten mit 1 bis 4 Kohlenstoffatomen, den Dialkylaminoresten, bei denen jeder Alkylteil 1 bis 4 Kohlenstoffatome enthält, dem Piperidino-, Morpholino-, 1-Piperazinyl-Rest (gegebenenfalls in 4-Stellung mit einem Alkylrest, der 1 bis 4 Kohlenstoffatome enthält, oder mit einem Phenylalkylrest, dessen Alkylteil 1 bis 4 Kohlenstoffatome enthält, substituiert), den Cycloalkylresten mit 3 bis 6 Kohlenstoffatomen, den Cycloalkenylresten mit 4 bis 6 Kohlenstoffatomen, dem Phenylrest (gegebenenfalls mit einem oder mehreren Atomen oder Resten substituiert, die unter den Halogenatomen und den Alkylresten mit 1 bis 4 Kohlenstoffatomen oder den Alkoxyresten mit 1 bis 4 Kohlenstoffatomen ausgewählt sind), dem Cyanorest, dem Carboxyrest oder dem Alkoxycarbonylrest, dessen Alkylteil 1 bis 4 Kohlenstoffatome enthält, ausgewählt sind,
- einen Phenyl- oder α- oder β-Naphthylrest, der gegebenenfalls mit einem oder mehreren Atomen oder Resten substituiert ist, die unter den Halogenatomen und den Alkylresten mit 1 bis 4 Kohlenstoffatomen oder den Alkoxyresten mit 1 bis 4 Kohlenstoffatomen oder einem aromatischen heterocyclischen Rest mit 5 Kettengliedern, der vorzugsweise unter dem Furyl- und Thienylrest ausgewählt ist, ausgewählt sind,
- oder einen gesättigten heterocyclischen Rest mit 4 bis 6 Kohlenstoffatomen, der gegebenenfalls mit einem oder mehreren Alkylresten mit 1 bis 4 Kohlenstoffatomen substituiert ist,

$R_3$ einen geraden oder verzweigten Alkylrest mit 1 bis 8 Kohlenstoffatomen, einen geraden oder verzweigten Alkenylrest mit 2 bis 8 Kohlenstoffatomen, einen geraden oder verzweigten Alkinylrest mit 2 bis 8 Kohlenstoffatomen, einen Cycloalkylrest mit 3 bis 6 Kohlenstoffatomen, einen Phenyl- oder α- oder β-Naphthylrest, der gegebenenfalls mit einem oder mehreren Atomen oder Resten substituiert ist, die unter den Halogenatomen und den Alkyl-, Alkenyl-, Alkinyl-, Aryl-, Aralkyl-, Alkoxy-, Alkylthio-, Aryloxy-, Arylthio-, Hydroxy-, Hydroxyalkyl-, Mercapto-, Formyl-, Acyl-, Acylamino-, Aroylamino-, Alkoxycarbonylamino-, Amino-, Alkylamino-, Dialkylamino-, Carboxy-, Alkoxycarbonyl-, Carbamoyl-, Alkylcarbamoyl-, Dialkylcarbamoyl-, Cyano-, Nitro- und Trifluormethylresten ausgewählt sind, oder einen aromatischen Heterocyclus mit 5 Kettengliedern, der ein oder mehrere, gleiche oder verschiedene, Heteroatome enthält, die unter den Stickstoff-, Sauerstoff- oder Schwefelatomen ausgewählt sind, und der gegebenenfalls mit einem oder mehreren, gleichen oder verschiedenen, Substituenten substituiert ist, die unter den Halogenatomen und den Alkyl-, Aryl-, Amino-, Alkylamino-, Dialkylamino-, Alkoxycarbonylamino-, Acyl-, Arylcarbonyl-, Cyano-, Carboxy-, Carbamoyl-, Alkylcarbamoyl-, Dialkylcarbamoyl- oder Alkoxycarbonylresten ausgewählt sind, darstellt, wobei es sich versteht, daß in den Substituenten der Phenyl-, α- oder β-Naphthylreste und der aromatischen heterocyclischen Reste, die Alkylreste und die Alkylteile der anderen Reste 1 bis 4 Kohlenstoffatome enthalten und daß die Alkenyl- und Alkinylreste 2 bis 8 Kohlenstoffatome enthalten und daß die Arylreste Phenyl- oder α- oder β-Naphthylreste sind.

**2.** Neue Taxoide gemäß Anspruch 1, für die R einen Alkylrest mit 1 bis 4 Kohlenstoffatomen darstellt, Z ein Wasserstoffatom oder einen Rest der allgemeinen Formel (II) darstellt, in der $R_1$ einen Benzoylrest oder einen Rest $R_2$-O-CO- darstellt, in dem $R_2$ einen tert.-Butylrest darstellt, und $R_3$ einen Alkylrest mit 1 bis 6 Kohlenstoffatomen, einen Alkenylrest mit 2 bis 6 Kohlenstoffatomen, einen Cycloalkylrest mit 3 bis 6 Kohlenstoffatomen, einen Phenylrest, gegebenenfalls substituiert mit einem oder mehreren, gleichen oder verschiedenen Atomen oder Resten, ausgewählt unter den Halogenatomen (Fluor, Chlor) und den Alkyl-, Alkoxy-, Dialkylamino-, Acylamino-, Alkoxycarbonylamino- - oder Trifluormethylresten, oder einen 2- oder 3-Furyl, 2- oder 3-Thienyl- oder 2-, 4- oder 5-Thiazolylrest darstellt.

**3.** Neue Taxoide gemäß Anspruch 1, für die R einen Methylrest darstellt, Z ein Wasserstoffatom oder einen Rest der allgemeinen Formel (II) darstellt, in der $R_1$ einen Benzoylrest oder einen Rest $R_2$-O-CO- darstellt, in dem $R_2$ einen tert.-Butylrest darstellt, und $R_3$ einen Isobutyl-, Isobutenyl-, Butenyl-, Cyclohexyl-, Phenyl-, 2-Furyl-, 3-Furyl-, 2-Thienyl-, 3-Thienyl-, 2-Thiazolyl-, 4-Thiazolyl- oder 5-Thiazolylrest darstellt.

**4.** Verfahren zur Herstellung eines neuen Taxoids gemäß einem der Ansprüche 1 bis 3, dadurch gekennzeichnet, daß man ein metallorganisches Derivat der allgemeinen Formel:

R-X (III)

in der R wie vorhergehend definiert ist und X ein Metallatom, wie ein Lithiumatom, oder einen magnesiumorganischen Rest darstellt, mit einem Produkt der allgemeinen Formel:

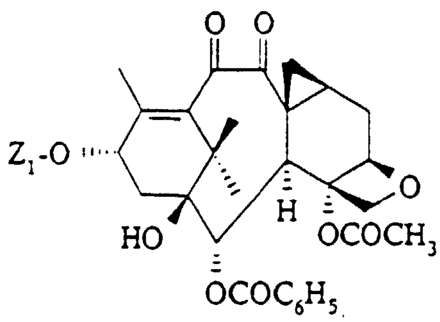

(IV)

in der $Z_1$ ein Wasserstoffatom oder einen Rest der allgemeinen Formel:

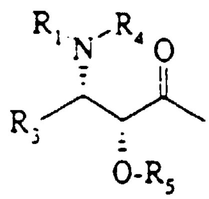

(V)

darstellt, in der $R_1$ und $R_3$ wie in einem der Ansprüche 1 bis 3 definiert sind, oder aber $R_4$ ein Wasserstoffatom darstellt und $R_5$ eine Schutzgruppe für die Hydroxyfunktion darstellt, und oder aber $R_4$ und $R_5$ zusammen einen Heterocyclus bilden, umsetzt, um ein Produkt der allgemeinen Formel:

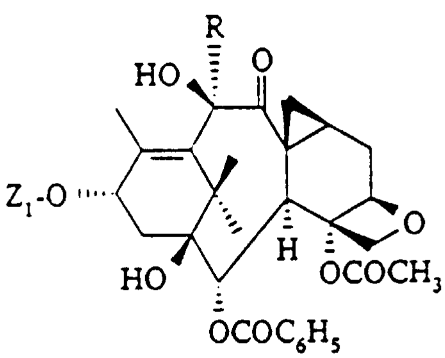

(VI)

zu erhalten, dann gegebenenfalls die Schutzgruppen, die von $R_5$ und/oder $R_4$ und $R_5$ dargestellt werden, durch Wasserstoffatome ersetzt.

**5.** Verfahren gemäß Anspruch 4, dadurch gekennzeichnet, daß man das Metall-Derivat umsetzt, indem man in einem inerten organischen Lösungsmittel bei einer Temperatur zwischen -78 und +30 °C arbeitet.

**6.** Verfahren gemäß Anspruch 4, dadurch gekennzeichnet, daß das Ersetzen der Schutzgruppen $R_5$ und/oder $R_4$ und $R_5$ auf die folgende Weise ausgeführt wird:

1) wenn $R_4$ ein Wasserstoffatom darstellt und $R_5$ eine Schutzgruppe für die Hydroxyfunktion darstellt, vollzieht

sich das Ersetzen der Schutzgruppen durch Wasserstoffatome mittels einer mineralischen oder organischen Säure, die allein oder im Gemisch verwendet wird, wobei man in einem organischen Lösungsmittel, das unter den Alkoholen, den Ethern, den Estern, den aliphatischen Kohlenwasserstoffen, den halogenierten aliphatischen Kohlenwasserstoffen, den aromatischen Kohlenwasserstoffen oder den Nitrilen ausgewählt ist, bei einer Temperatur zwischen -10 und 60 °C arbeitet,
2) wenn $R_4$ und $R_5$ zusammen einen Oxazolidin-Ring der allgemeinen Formel:

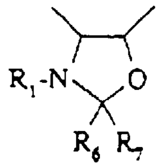

(VII)

bilden, in der $R_1$ wie in einem der Ansprüche 1 bis 3 definiert ist, $R_6$ und $R_7$, gleich oder verschieden, ein Wasserstoffatom oder einen Alkylrest mit 1 bis 4 Kohlenstoffatomen oder einen Aralkylrest darstellen, dessen Alkylteil 1 bis 4 Kohlenstoffatome enthält und dessen Arylteil vorzugsweise einen Phenylrest, der gegebenenfalls mit einem oder mehreren Alkoxyresten mit 1 bis 4 Kohlenstoffatomen substituiert ist, oder einen Arylrest, vorzugsweise einen Phenylrest, der gegebenenfalls mit einem oder mehreren Alkoxyresten mit 1 bis 4 Kohlenstoffatomen substituiert ist, darstellt, oder aber $R_6$ einen Alkoxyrest mit 1 bis 4 Kohlenstoffatomen oder einen Trihalogenmethylrest, wie einen Trichlormethylrest, oder einen mit einem Trihalogenmethylrest, wie einem Trichlormethylrest, substituierten Phenylrest darstellt und $R_7$ ein Wasserstoffatom darstellt, oder aber $R_6$ und $R_7$ zusammen mit dem Kohlenstoffatom, mit dem sie verbunden sind, einen Ring mit 4 bis 7 Kettengliedern bilden, kann das Ersetzen der von $R_6$ und $R_7$ gebildeten Schutzgruppe durch Wasserstoffatome je nach den Bedeutungen von $R_1$, $R_6$ und $R_7$ auf die folgende Weise ausgeführt werden:

a) wenn $R_1$ einen tert.-Butoxycarbonylrest darstellt, $R_6$ und $R_7$, gleich oder verschieden, einen Alkylrest oder einen Aralkyl- oder Arylrest darstellen, oder aber $R_6$ einen Trihalogenmethylrest oder einen mit einem Trihalogenmethylrest substituierten Phenylrest darstellt und $R_7$ ein Wasserstoffatom darstellt, oder aber $R_6$ und $R_7$ zusammen einen Ring mit 4 bis 7 Kettengliedern bilden, führt die Behandlung des Esters der allgemeinen Formel (VI) mit einer mineralischen oder organischen Säure gegebenenfalls in einem organischen Lösungsmittel, wie einem Alkohol, zu dem Produkt der allgemeinen Formel:

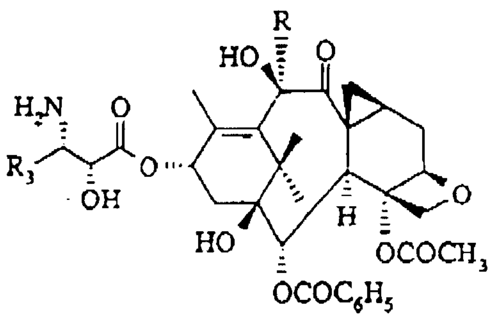

(VIII)

in der R und $R_3$ wie in einem der Ansprüche 1 bis 3 definiert sind, das mittels Benzoylchlorid, in dem der Phenylkern gegebenenfalls substituiert ist, Thenoylchlorid, Furoylchlorid oder einem Produkt der allgemeinen Formel:

$$R_2\text{-O-CO-X} \qquad (IX)$$

in der $R_2$ wie vorhergehend definiert ist und X ein Halogenatom (Fluor, Chlor) oder einen Rest $-O-R_2$ oder $-O-CO-O-R_2$ darstellt, acyliert wird, um ein Produkt der allgemeinen Formel (I), in der Z einen Rest der allgemeinen Formel (II) darstellt, zu erhalten,

b) wenn $R_1$ einen gegebenenfalls substituierten Benzoylrest, einen Thenoyloder Furylrest oder einen Rest $R_2O$-CO- darstellt, in dem $R_2$ wie in einem der Ansprüche 1 bis 3 defniert ist, $R_6$ ein Wasserstoffatom oder einen Alkoxyrest mit 1 bis 4 Kohlenstoffatomen oder einen mit einem oder mehreren Alkoxyresten mit 1 bis 4 Kohlenstoffatomen substituierten Phenylrest darstellt und $R_7$ ein Wasserstoffatom darstellt, vollzieht sich das Ersetzen der von $R_6$ und $R_7$ gebildeten Schutzgruppe durch Wasserstoffatome in Gegenwart einer mineralischen oder organischen Säure, die allein oder im Gemisch in stöchiometrischer oder katalytischer Menge verwendet wird, wobei man in einem organischen Lösungsmittel, das unter den Alkoholen, den Ethern, den Estern, den aliphatischen Kohlenwasserstoffen, den halogenierten aliphatischen Kohlenwasserstoffen und den aromatischen Kohlenwasserstoffen ausgewählt ist, bei einer Temperatur zwischen -10 und 60 °C arbeitet.

**7.** Pharmazeutische Zusammensetzung, dadurch gekennzeichnet, daß sie wenigstens ein Produkt gemäß einem der Ansprüche 1 bis 3, für das Z einen Rest der allgemeinen Formel (II) darstellt, in Verbindung mit einem oder mehreren pharmazeutisch geeigneten Produkten, mögen sie inert oder pharmakologisch aktiv sein, enthält.

## Claims

**1.** New taxoids of general formula:

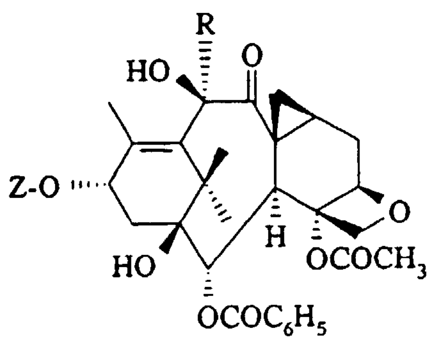

(I)

in which:

- R represents an unbranched or branched alkyl radical containing 1 to 6 carbon atoms, an unbranched or branched alkenyl radical containing 2 to 6 carbon atoms, an unbranched or branched alkynyl radical containing 2 to 6 carbon atoms, a cycloalkyl radical containing 3 to 6 carbon atoms, a cycloalkenyl radical containing 4 to 6 carbon atoms, an aryl radical or a 5- to 6-membered aromatic heterocyclic radical,

Z represents a hydrogen atom or a radical of general formula:

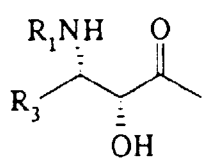

(II)

in which:

$R_1$ represents a benzoyl radical optionally substituted with one or more identical or different atoms or radicals chosen from halogen atoms and alkyl radicals containing 1 to 4 carbon atoms, alkoxy radicals containing 1 to 4 carbon atoms or trifluoromethyl radicals, a thenoyl or furoyl radical or a radical $R_2$-OCO- in which $R_2$ represents:

- an alkyl radical containing 1 to 8 carbon atoms, an alkenyl radical containing 2 to 8 carbon atoms, an

alkynyl radical containing 3 to 8 carbon atoms, a cycloalkyl radical containing 3 to 6 carbon atoms, a cycloalkenyl radical containing 4 to 6 carbon atoms or a bicycloalkyl radical containing 7 to 10 carbon atoms, these radicals being optionally substituted with one or more substituents chosen from halogen atoms and hydroxyl radicals, alkoxy radicals containing 1 to 4 carbon atoms, dialkylamino radicals in which each alkyl portion contains 1 to 4 carbon atoms, piperidino or morpholino radicals, 1-piperazinyl radicals (optionally substituted at position 4 with an alkyl radical containing 1 to 4 carbon atoms or with a phenylalkyl radical in which the alkyl portion contains 1 to 4 carbon atoms), cycloalkyl radicals containing 3 to 6 carbon atoms, cycloalkenyl radicals containing 4 to 6 carbon atoms, phenyl radicals (optionally substituted with one or more atoms or radicals chosen from halogen atoms and alkyl radicals containing 1 to 4 carbon atoms or alkoxy radicals containing 1 to 4 carbon atoms), cyano or carboxyl radicals or alkoxycarbonyl radicals in which the alkyl portion contains 1 to 4 carbon atoms,

- a phenyl or α- or β-naphthyl radical optionally substituted with one or more atoms or radicals chosen from halogen atoms and alkyl radicals containing 1 to 4 carbon atoms or alkoxy radicals containing 1 to 4 carbon atoms, or a 5-membered aromatic heterocyclic radical preferably chosen from furyl and thienyl radicals,
- or a saturated heterocyclic radical containing 4 to 6 carbon atoms, optionally substituted with one or more alkyl radicals containing 1 to 4 carbon atoms,

$R_3$ represents an unbranched or branched alkyl radical containing 1 to 8 carbon atoms, an unbranched or branched alkenyl radical containing 2 to 8 carbon atoms, an unbranched or branched alkynyl radical containing 2 to 8 carbon atoms, a cycloalkyl radical containing 3 to 6 carbon atoms, a phenyl or α- or β-naphthyl radical optionally substituted with one or more atoms or radicals chosen from halogen atoms and alkyl, alkenyl, alkynyl, aryl, aralkyl, alkoxy, alkylthio, aryloxy, arylthio, hydroxyl, hydroxyalkyl, mercapto, formyl, acyl, acylamino, aroylamino, alkoxycarbonylamino, amino, alkylamino, dialkylamino, carboxyl, alkoxycarbonyl, carbamoyl, alkylcarbamoyl, dialkylcarbamoyl, cyano, nitro and trifluoromethyl radicals, or a 5-membered aromatic heterocycle containing one or more identical or different hetero atoms chosen from nitrogen, oxygen and sulphur atoms and optionally substituted with one or more identical or different substituents chosen from halogen atoms and alkyl, aryl, amino, alkylamino, dialkylamino, alkoxycarbonylamino, acyl, arylcarbonyl, cyano, carboxyl, carbamoyl, alkylcarbamoyl, dialkylcarbamoyl or alkoxycarbonyl radicals, on the understanding that, in the substituents of the phenyl, α or β-naphthyl and aromatic hetereocyclic radicals, the alkyl radicals and the alkyl portions of the other radicals contain 1 to 4 carbon atoms, and that the alkenyl and alkynyl radicals contain 2 to 8 carbon atoms, and that the aryl radicals are phenyl or α- or β-naphthyl radicals.

**2.** New taxoids according to claim 1 for which R represents an alkyl radical containing 1 to 4 carbon atoms, Z represents a hydrogen atom or a radical of general formula (II) in which $R_1$ represents a benzoyl radical or a radical $R_2$-O-CO- in which $R_2$ represents a tert-butyl radical and $R_3$ represents an alkyl radical containing 1 to 6 carbon atoms, an alkenyl radical containing 2 to 6 carbon atoms, a cycloalkyl radical containing 3 to 6 carbon atoms, a phenyl radical optionally substituted with one or more identical or different atoms or radicals chosen from halogen atoms (fluorine, chlorine) and alkyl, alkoxy, dialkylamino, acylamino, alkoxycarbonylamino or trifluoromethyl radicals, or a 2- or 3-furyl, 2- or 3-thienyl or 2-, 4- or 5-thiazolyl radical.

**3.** New taxoids according to claim 1 for which R represents a methyl radical, Z represents a hydrogen atom or a radical of general formula (II) in which $R_1$ represents a benzoyl radical or a radical $R_2$-O-CO- in which $R_2$ represents a tert-butyl radical and $R_3$ represents an isobutyl, isobutenyl, butenyl, cyclohexyl, phenyl, 2furyl, 3-furyl, 2-thienyl, 3-thienyl, 2-thiazolyl, 4thiazolyl or 5-thiazolyl radical.

**4.** Process for preparing a new taxoid according to one of claims 1 to 3, characterized in that an organometallic derivative of general formula:

$$R\text{-}X \qquad \text{(III)}$$

in which R is defined as above and X represents a metal atom such as a lithium atom or an organomagnesium residue, is reacted with a product of general formula:

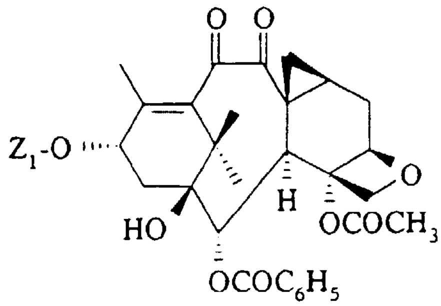

(IV)

in which $Z_1$ represents a hydrogen atom or a radical of general formula:

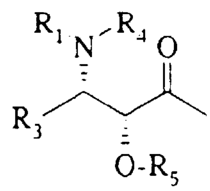

(V)

in which $R_1$ and $R_3$ are defined as in one of claims 1 to 3, and either $R_4$, represents a hydrogen atom and $R_5$ represents a group protecting the hydroxyl function, or $R_4$, and $R_5$ together form a heterocycle, to obtain a product of general formula:

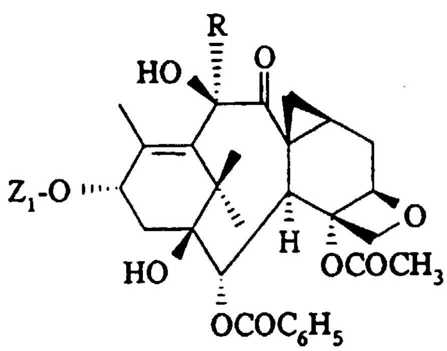

(VI)

and the protective groups represented by $R_5$ and/or $R_4$, and $R_5$ are then, where appropriate, replaced by hydrogen atoms.

**5.** Process according to claim 4, characterized in that the metal derivative is reacted by working in an inert organic solvent at a temperature of between -78 and +30°C.

**6.** Process according to claim 4, characterized in that replacement of the protective groups $R_5$ and/or $R_4$, and $R_5$ is performed in the following manner:

1) when $R_4$, represents a hydrogen atom and $R_5$ represents a group protecting the hydroxyl function, replacement of the protective groups by hydrogen atoms is performed by means of an inorganic or organic acid used alone or mixed, working in an organic solvent chosen from alcohols, ethers, esters, aliphatic hydrocarbons, halogenated aliphatic hydrocarbons, aromatic hydrocarbons or nitriles at a temperature of between -10 and 60°C,
2) when $R_4$, and $R_5$ together form an oxazolidine ring of general formula:

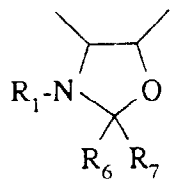

(VII)

in which $R_1$ is defined as in one of claims 1 to 3 and $R_6$ and $R_7$, which may be identical or different, represent a hydrogen atom or an alkyl radical containing 1 to 4 carbon atoms, or an aralkyl radical in which the alkyl portion contains 1 to 4 carbon atoms and the aryl portion preferably represents a phenyl radical optionally substituted with one or more alkoxy radicals containing 1 to 4 carbon atoms, or an aryl radical preferably representing a phenyl radical optionally substituted with one or more alkoxy radicals containing 1 to 4 carbon atoms, or alternatively $R_6$ represents an alkoxy radical containing 1 to 4 carbon atoms or a trihalomethyl radical such as trichloromethyl or a phenyl radical substituted with a trihalomethyl radical such as trichloromethyl and $R_7$ represents a hydrogen atom, or alternatively $R_6$ and $R_7$, together with the carbon atom to which they are attached, form a 4- to 7-membered ring, replacement of the protective group formed by $R_6$ and $R_7$ by hydrogen atoms may be performed, depending on the meanings of $R_1$, $R_6$ and $R_7$, in the following manner:

a) when $R_1$ represents a tert-butoxycarbonyl radical and $R_6$ and $R_7$, which may be identical or different, represent an alkyl radical or an aralkyl or aryl radical, or alternatively $R_6$ represents a trihalomethyl radical or a phenyl radical substituted with a trihalomethyl radical and $R_7$ represents a hydrogen atom, or alternatively $R_6$ and $R_7$ together form a 4- to 7-membered ring, treatment of the ester of general formula (VI) with an inorganic or organic acid, where appropriate in an organic solvent such as an alcohol, yields the product of general formula:

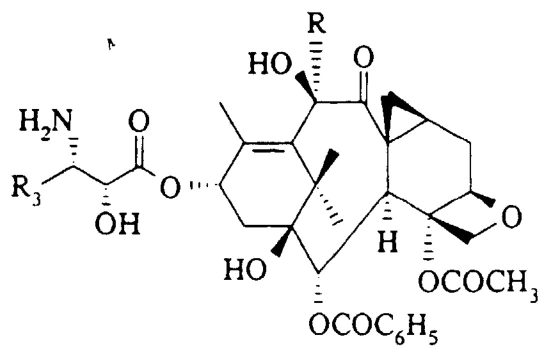

(VIII)

in which R and $R_3$ are defined as in one of claims 1 to 3, which is acylated by means of benzoyl chloride in which the phenyl ring is optionally substituted or by means of thenoyl chloride, of furoyl chloride or of a product of general formula:

$$R_2\text{-O-CO-X} \qquad (IX)$$

in which $R_2$ is defined as above and X represents a halogen atom (fluorine, chlorine) or a residue -O-$R_2$ orO-CO-O-$R_2$, to obtain a product of general formula (I) in which Z represents a radical of general formula (II),

b) when $R_1$ represents an optionally substituted benzoyl radical, a thenoyl or furoyl radical or a radical $R_2$O-CO- in which $R_2$ is defined as in one of claims 1 to 3, $R_6$ represents a hydrogen atom or an alkoxy radical containing 1 to 4 carbon atoms or a phenyl radical substituted with one or more alkoxy radicals containing 1 to 4 carbon atoms and $R_7$ represents a hydrogen atom, replacement of the protective group formed by $R_6$ and $R_7$ by hydrogen atoms is performed in the presence of an inorganic or organic acid used alone or mixed in a stoichiometric or catalytic amount, working in an organic solvent chosen from alcohols, ethers, esters, aliphatic hydrocarbons, halogenated aliphatic hydrocarbons and aromatic hydrocarbons at a temperature of between -10 and 60°C.

7. Pharmaceutical composition, characterized in that it contains at least one product according to one of claims 1 to 3 for which Z represents a radical of general formula (II), in combination with one or more pharmaceutically acceptable products which may be inert or pharmacologically active.